# EUROPÄISCHE PATENTSCHRIFT

(11) **EP 2 528 651 B1**
(45) Veröffentlichungstag und Bekanntmachung des Hinweises auf die Patenterteilung: **19.03.2014**
(21) Anmeldenummer: 11700790.6
(22) Anmeldetag: 05.01.2011
(51) Int. Cl.: A61M 27/00

(54) **ANSCHLUSSVORRICHTUNG ZUR VERWENDUNG BEI DER UNTERDRUCKBEHANDLUNG VON WUNDEN**
CONNECTION DEVICE FOR USE IN NEGATIVE-PRESSURE WOUND THERAPY
DISPOSITIF DE LIAISON EMPLOYÉ POUR LE TRAITEMENT DE PLAIES PAR PRESSION NÉGATIVE

(30) Priorität: 26.01.2010 DE 102010006273
(43) Veröffentlichungstag der Anmeldung: 05.12.2012
(73) Patentinhaber: Paul Hartmann Aktiengesellschaft, 89522 Heidenheim (DE)
(72) Erfinder: CROIZAT, Pierre, 89542 Herbrechtingen (DE); ECKSTEIN, Axel, 89522 Heidenheim (DE); HOFSTETTER, Jürgen, 89522 Heidenheim (DE)
(74) Vertreter: DREISS Patentanwälte PartG mbB
(86) Internationale Anmeldenummer: PCT/EP2011/000009
(87) Internationale Veröffentlichungsnummer: WO 2011/091947

(56) Entgegenhaltungen:
- WO-A1-2009/002260
- WO-A2-2008/048527
- US-A1- 2007 219 497
- US-A1- 2009 234 307

## Beschreibung

Die Erfindung betrifft eine Anschlussvorrichtung zur Verwendung bei der Unterdruckbehandlung von Wunden, mit einem mit Unterdruck beaufschlagbaren Leitungsmittel und mit einem flächenhaften unterdruckdichten ersten Trägermittel für das Leitungsmittel, an dem das Leitungsmittel unterdruckdicht gehalten ist, wobei das Trägermittel auf einen die Wunde überfangenden und gegen die Atmosphäre dichtend abschließenden Unterdruckverband aufbringbar ist, wobei das Leitungsmittel durch wenigstens eine Öffnung in der dem Unterdruckverband zugewandten Wandung des Leitungsmittels und durch wenigstens eine Öffnung in dem Unterdruckverband hindurch mit dem Wundraum kommuniziert.

In der jüngeren Vergangenheit hat die Unterdruckbehandlung von Wunden, insbesondere von tiefen und daher a priori problematisch heilenden Wunden, eine zunehmende Bedeutung erlangt. Dabei bedeutet Unterdruckbehandlung, dass ein an sich der umgebenden Atmosphäre ausgesetzter Körper- oder Wundbereich durch noch näher zu beschreibende Mittel druckdicht bzw. unterdruckdicht gegen die Umgebung, also die Atmosphäre in der wir leben und atmen, abgeschlossen wird, wobei innerhalb des abgeschlossenen Wundbereichs auf ebenfalls noch zu erläuternde Weise ein gegenüber dem Atmosphärendruck verringerter Druck, mithin also Unterdruck relativ zur Atmosphäre angelegt und dauerhaft aufrechterhalten werden kann. Wenn auf dem hier in Rede stehenden Gebiet von Unterdruck die Rede ist, so wird hiermit ein Druckbereich verstanden, der typischerweise zwischen 0 und 500 mm Hg (mm Quecksilbersäule) unterhalb des umgebenden Atmosphärendrucks liegt. Es hat sich gezeigt, dass dies der Wundheilung förderlich ist. Für den unterdruckdichten Abschluss wird ein Unterdruckverband vorgesehen, der beispielsweise eine druck- bzw. unterdruckdichte Folienschicht umfassen kann, die typischerweise mit einem die Wunde umgebenden unversehrten Körperbereich verklebt ist, so dass auf diese Weise ein dichter Abschluss erreicht werden kann. Um ausgehend von einer Unterdruck erzeugenden Einrichtung, also einer Unterdruckpumpe im weitesten Sinn, einen Unterdruck an den Wundraum heranzuführen und dort aufrechtzuerhalten, können bei hier in Rede stehenden Systemen zur Unterdrucktherapie von Wunden mit Unterdruck beaufschlagbare Leitungsmittel verwendet werden, die mittels einer Anschlussvorrichtung mit dem Unterdruckverband zusammenwirken, um Unterdruck an bzw. in den Wundraum zu bringen. Eine derartige Anschlussvorrichtung der eingangs erwähnten Art ist beispielsweise aus WO 2006/052338 A2 bekannt. Es werden röhrenförmige Schläuche verwandt, um den Unterdruck an die Wunde heranzuführen. US 2009/0234307 A1 zeigt eine Anschlussvorrichtung zur Verwendung bei der Unterdruckbehandlung von Wunden, mit einem mit Unterdruck beaufschlagbaren biegsamen rundschlauchartigen Leitungsmittel und mit einem flächenhaften unterdruckdichten ersten Trägermittel für das Leitungsmittel, an dem das Leitungsmittel unterdruckdicht gehalten ist, wobei das Trägermittel auf einen die Wunde überfangenden und gegen die Atmosphäre dichtend abschließenden Unterdruckverband aufbringbar ist, wobei das Leitungsmittel einen Längsabschnitt aufweist, an dessen Ende eine Öffnung vorgesehen ist. Das Trägermittel ist von einem Flansch gebildet, von dem sich einstückig nach oben ein Einstecksegment erhebt, in welches das Leitungsmittel einsteckbar ist. Dieses Einstecksegment baut insgesamt recht hoch. WO 2008/048527 A2 zeigt bei einer Ausführungsform einen nicht gattungsgemäßen Verbund aus Anschlussvorrichtung und Unterdruckverband, bei dem also kein separates Trägermittel von dem Unterdruckverband unterschieden werden kann. Das Leitungsmittel ist hier quasi durch den Unterdruckverband hindurch in den Wundraum hineingeführt. Eine andere Ausführungsform zeigt ein schematisch dargestelltes flach bauendes Leitungsmittel, welches von außen flächenhaft an einen Unterdruckverband angefügt ist.

Der vorliegenden Erfindung liegt die Aufgabe zugrunde, eine Anschlussvorrichtung der eingangs beschriebenen Art zu schaffen, die sich für den Patienten als angenehm bzw. wenig schmerzauslösend bei Belastung oder Berührung erweist und bei der die Abdichtung der Komponenten mit technisch und wirtschaftlich vertretbarem Aufwand sowie bedienerfreundlich realisierbar ist.

Diese Aufgabe wird durch eine Anschlussvorrichtung mit den Merkmalen des Anspruchs 1 gelöst.

Das Leitungsmittel ist also nicht röhrenförmig mit im wesentlichen rundem Querschnitt ausgebildet, sondern es weist eine flache Form auf, dessen Breitenerstreckung wesentlich größer als seine Dickenerstreckung ist. Dies führt zusammen mit der Materialauswahl zu einem nachgiebigen Leitungsmittel, welches sich für den Patienten als angenehmer erweist, wenn auf die Anschlussvorrichtung oder das Leitungsmittel ein Berührungsdruck ausgeübt wird. Es kommt somit weniger zu einer punktuellen Belastung, die naturgemäß Schmerz auslösend sein kann und insbesondere bei schmerzempfindlichen frischen Wunden höchst problematisch ist. Außerdem besteht in Folge der flachen Ausbildung eine geringere Gefahr des Hinterhakens oder Hängenbleibens. Durch die flächenhafte Ausbildung des Leitungsmittels und des Trägermittels verteilt sich eine Druckbelastung auf einen großflächigeren Bereich des Wundverbands, was sich im Hinblick auf die vorstehend erörterte Problematik als sehr vorteilhaft erweist.

Dadurch, dass in dem ersten Trägermittel erfindungsgemäß eine ausladende Ausnehmung ausgebildet ist, in die sich das flache Leitungsmittel mit einem Längsabschnitt flächenhaft hineinerstreckt und somit durch diese Ausnehmung hindurch dem Unterdruckverband zugewandt ist ohne Zwischenordnung weiterer Materialien, oder gegen den Unterdruckverband anliegt, wird die Dickenerstreckung in diesem sensiblen Bereich der Anschlussvorrichtung weiter reduziert, was sich im Hinblick auf die vorausgehend geschilderte Problematik als vorteilhaft erweist.

Die ausladende Ausnehmung in dem Trägermittel könnte zwar grundsätzlich randoffen ausgebildet sein, so dass sich das Leitungsmittel kontinuierlich von außen in diese Ausnehmung hineinerstrecken kann. Aufgrund der sich hierbei stellenden Abdichtungsproblematik wird jedoch einer Ausführungsform der Vorzug gegeben, bei der die ausladende Öffnung in dem Trägermittel in Umfangsrichtung umgeben ist. Das Leitungsmittel erstreckt sich dann zunächst in flächenhafter Anlage an das ebenfalls flächenhafte Trägermittel, um sich dann weiter in die ausladende Ausnehmung in dem Trägermittel hineinzuerstrecken. Die Abdichtung gegenüber dem Trägermittel und der Atmosphäre, die nachfolgend noch im Einzelnen erörtert werden wird, gestaltet sich dann als einfacher realisierbar.

In weiterer Ausbildung des Erfindungsgedankens erweist es sich als vorteilhaft, wenn die Länge der Ausnehmung wenigstens 20 mm, insbesondere wenigstens 30 mm und weiter insbesondere wenigstens 40 mm beträgt.

Die Aufbringung bzw. Anhaftung der erfindungsgemäßen Anschlussvorrichtung an die wundabgewandte Oberseite des Unterdruckverbands kann in an sich beliebiger Weise, also beispielsweise durch Haftvermittler oder durch zusätzliche tape- oder folienartige Mittel erfolgen. Bei einer bevorzugten Ausführungsform der Erfindung trägt das erste Trägermittel auf seiner dem Unterdruckverband zugewandten Seite eine Kleberbeschichtung oder es ist dort selbsthaftend ausgebildet, so dass die Anschlussvorrichtung direkt auf eine wundabgewandte Oberseite des Unterdruckverbands anhaftbar und unterdruckdicht applizierbar ist. Das Trägermittel und dessen Kleberbeschichtung realisieren nach dieser Ausführungsform also die vorzugsweise lösbare Haftverbindung zu dem Unterdruckverband.

In weiterer Ausbildung der Erfindung erweist es sich als vorteilhaft, dass das Leitungsmittel dadurch unterdruckdicht an dem ersten Trägermittel gehalten ist, dass es von dem zweiten flächenhaften Trägermittel überfangen und so zwischen dem ersten und dem zweiten Trägermittel sandwichartig aufgenommen ist, wobei das zweite Trägermittel in der Projektion auf die Ebene des ersten Trägermittels die Ausnehmung vollständig überfängt. Es hat sich gezeigt, dass nach diesem weiteren Erfindungsgedanken eine auf wirtschaftliche Weise herstellbare und insgesamt wenig auftragende Abdichtung des Leitungsmittels gegenüber dem Trägermittel erreicht werden kann. Es stehen nach diesem Erfindungsgedanken flächenhaft überlappende, also flächenhaft gegeneinander anliegende biegsame Flachmaterialien zur Verfügung, die aufgrund ihrer flächenhaften Anlage gut gegeneinander abdichtbar sind.

Es erweist sich hierbei als vorteilhaft, wenn das zweite Trägermittel in Bezug auf die Erstreckung des ersten Trägermittels im Bereich der Ausmündung des Leitungsmittels zurückgesetzt ist, so dass dort ein überlappungsfreier Bereich des ersten Leitungsmittels gebildet ist.

Zum Abdichten, insbesondere im Ausmündungsbereich des Leitungsmittels zwischen den Trägermitteln, könnten die Materialien unmittelbar, insbesondere thermisch nach Art einer Vulkanisierverbindung miteinander gefügt werden, oder es wäre denkbar, dass zum Abdichten eine Dichtmasse, insbesondere auf Silikonbasis, verwandt ist. Es kann sich als hinreichend erweisen, wenn die Dichtmasse nur im Ausmündungsbereich des Leitungsmittels zwischen den Trägermitteln vorgesehen ist und dabei den Bereich um das Leitungsmittel herum abdichten. Die Dichtmasse kann zugleich haftvermittelnd zwischen dem ersten und dem zweiten Trägermittel wirken.

Weiter erweist es sich als vorteilhaft, wenn das erste und/oder das zweite Trägermittel aus einem thermoplastischen Elastomer, insbesondere aus Silikon oder aus Polyurethan oder bevorzugt aus einem Hydrokolloidmaterial, insbesondere aus einem selbsthaftenden Hydrokolloidmaterial, gebildet sind oder solche Materialien umfassen. Dabei wird einem Hydrokolloidmaterial der Vorzug gegeben. Unter einem Hydrokolloidmaterial wird eine Matrixmasse mit darin homogen dispergierten Hydrokolloiden (z.B. Natrium-Carboxymethylcellulose) verstanden. Unter einem selbsthaftenden Hydrokolloidmaterial wird eine druckempfindliche Haftklebemasse (z.B. auf Basis eines synthetischen Kautschuks) mit darin homogen dispergierten Hydrokolloiden (z.B. Natrium-Carboxymethylcellulose) verstanden.

Beispielsweise können zur Verwendung als Wundauflagen geeignete Hydrokolloid-Flachmaterialien von der Firma Avery Dennison (Turnhout, Belgien) unter dem Handelsnamen MED 5598H erhalten werden. Die flächenhaften Trägermittel, insbesondere mit oder aus Hydrokolloidmaterial, können einen Trägerfilm, beispielsweise einen Polyurethanfilm und/oder eine ablösbare Release-Beschichtung umfassen. Die vorerwähnten Hydrokolloid-Flachmaterialien haben auf einer Seite einen Polyurethanfilm und gegenüber eine ablösbare Release-Beschichtung.

Das elastomere Material, aus dem das Leitungsmittel gebildet ist, weist vorzugsweise eine Shore A - Härte von Höchstens 60, insbesondere von 5 - 60, insbesondere von 10 - 60, insbesondere von 15 - 50, insbesondere von 15 - 40 und weiter insbesondere von 15 - 35 auf. Die Shore - A Härte wird nach DIN 53505 vom August 2000 bestimmt, und zwar bei 23°C an einem wie in der Norm beschrieben plattenförmigen ebenen und glatten Probekörper einer Dicke von 6 mm. Nach einer bevorzugten Ausführungsform der Erfindung ist das Leitungsmittel auf Silikonbasis ausgebildet.

Es wurde bereits darauf hingewiesen, dass die erfindungsgemäße Anschlussvorrichtung senkrecht zur Ebene des Unterdruckverbands sehr flach baut. Diese Dickenerstreckung des Verbunds aus Leitungsmittel und Trägermittel(n) beträgt in bevorzugter Weise höchstens 10 mm, insbesondere höchstens 7 mm, insbesondere höchstens 6 mm und weiter insbesondere höchstens 5 mm.

Die Breitenerstreckung des flachen Leitungsmittels beträgt wenigstens 10 mm, insbesondere wenigstens 15 mm und weiter insbesondere wenigstens 18 mm und insbesondere höchstens 30 mm und weiter insbesondere höchstens 25 mm beträgt.

Da das flache und biegsam ausgebildete Leitungsmittel zum Heranführen von Unterdruck in den Wundraum und gegebenenfalls zum Heranführen von Spülflüssigkeiten oder Spülgasen und dem Abführen von Wundsekreten dient, also vorzugsweise lediglich eine kanalbildende Kommunikationsfunktion hat, wird vorgeschlagen, das Leitüngsmittel nicht laminatartig mit mehreren Komponenten oder Schichten auszubilden, sondern trotz seiner flachbauenden Form schlauchförmig, das heißt in einem Querschnitt betrachtet in Umfangsrichtung einstückig durchgehend aus einem einzigen Material auszubilden.

Weiter erweist es sich als vorteilhaft, wenn das Leitungsmittel im Inneren angeformte, insbesondere mit dem Material des Leitungsmittels einstückig ausgebildete Mittel zur Verhinderung des Kollabierens des Leitungsmittels bei Unterdruckbeaufschlagung aufweist. Diese Mittel zur Verhinderung des Kollabierens des Leitungsmittel können gerade bei einem im vorstehend geschilderten Sinn schlauchförmigen Leitungsmittel vorgesehen werden. Diese Mittel zur Verhinderung des Kollabierens können beispielsweise von Rippen oder Vorsprüngen gebildet sein. In Weiterbildung dieses Erfindungsgedankens erweist es sich als vorteilhaft, wenn sie kontinuierlich durchgehend erstreckt sind. Das Leitungsmittel kann dann in vorteilhafter Weise als Extrusionsteil ausgebildet sein.

Es kann sich ferner als vorteilhaft erweisen, wenn das Leitungsmittel mehrere voneinander druckdicht abgetrennte Kanäle umfasst, wobei das Leitungsmittel auch solchenfalls vorzugsweise einstückig ausgebildet ist, also keine Zusammenfassung von mehreren separaten kanalbildenden Mitteln aufweist. Die mehreren Kanäle können einen Spülkanal, der ein Spülmedium in Richtung auf das wundzugewandte Ende der Anschlussvorrichtung leiten kann, und einen Unterdruck führenden Kanal, der der Unterdruckzuführung bzw. der Abführung von Wundsekreten dient, umfassen. Hierdurch können auch etwaige Verstopfungen innerhalb des Leitungsmittels gelöst werden. Jeder Kanal kommuniziert dabei mit wenigstens einer Öffnung in dem Leitungsmittel.

Vorzugsweise erstreckt sich das flache Leitungsmittel über eine gewisse Distanz in Längsrichtung und kann dann über ein nicht dargestelltes Übergangs- oder Kopplungselement, welches eine Steckverbindung oder Klebeverbindung bilden kann, in einen üblichen verwindungssteiferen Rundschlauch übergehen, der zu einer Unterdruck erzeugenden Vorrichtung führt, die als stationäres Gerät oder als mobil am Körper des Patienten tragbares Gerät ausgebildet sein kann. Das Übergangs- oder Kopplungselement kann auch zum Koppeln eines mehrkanaligen Leitungsmittels mit einem mehrkanaligen Rundschlauch ausgebildet sein. Als zweckmäßige Längserstreckung des flachen Leitungsmittels hat sich eine Strecke von 10 - 60 cm erwiesen.

Das flächenhafte erste und/oder zweite Trägermittel der Anschlussvorrichtung, mit dem das flache Leitungsmittel herstellerseitig unterdruckdicht verbunden wird, ist vorzugsweise ebenfalls aus einem biegsamen elastomeren Material einer Shore A - Härte von 5 - 60, insbesondere von 10 - 60, insbesondere von 15 - 50, insbesondere von 15 - 40 und weiter insbesondere von 15 - 35 gebildet. Das flächenhafte Trägermittel weist vorteilhafterweise eine Dicke von 0,75 - 3 mm, insbesondere von 1 - 3 mm auf.

Das Trägermittel dient dazu, das flache Leitungsmittel zu halten und gleichmäßig zu stützen. Seine flächenhafte Erstreckung ist daher größer als die flächenhafte Erstreckung des Leitungsmittels in dem betreffenden wundseitigen Längsende. Es erweist sich insoweit als vorteilhaft, wenn die flächenhafte Erstreckung des ersten Trägermittels wenigstens das 1,5-Fache, vorzugsweise wenigstens das Doppelte der senkrecht auf das Trägermittel projizierten Fläche des Leitungsmittels umfasst, da hierdurch die über das Leitungsmittel bei Berührung eingeleiteten Kräfte über eine größere Fläche verteilt werden und auch Biegemomente, die auf das Leitungsmittel ausgeübt werden, nicht oder in geringerem Maße auf den Unterdruckverband übertragen werden; sie werden besser durch das plattenförmige Trägermittel aufgenommen. Es erweist sich als ausreichend, wenn das vorstehend erwähnte Flächenverhältnis höchstens 5, insbesondere höchstens 4 beträgt, wobei sich ein Verhältnis von 2 - 3 als vorteilhaft erwiesen hat.

Das flache Leitungsmittel könnte im Querschnitt betrachtet beispielsweise eine Rechteckform aufweisen, wobei die beiden Schmalseiten auch und vorzugsweise verrundet ausgebildet sein können. Nach einer weiteren Ausführungsform der Erfindung ist das Leitungsmittel im Querschnitt betrachtet trapezförmig ausgebildet. Die Schmalseiten fallen dann mit einem beispielhaften Neigungswinkel zur Ebene des flächenhaften Trägermittels von 25° bis 60°, insbesondere von 35° - 50° schräg ab, wobei die Flanken der einseitigen oder vorzugsweise zweiseitigen Trapezform nicht zwingend gerade, sondern durchaus auch verrundet verlaufen können.

Im Hinblick auf die Anzahl und die Größe der Öffnungen in der dem Unterdruckverband zugewandten Flachseite oder Wandung des Leitungsmittels wäre es an sich denkbar, dass nur eine einzige Öffnung vorgesehen ist, die mit einer korrespondierenden Öffnung in dem Unterdruckverband und somit mit dem Wundraum kommuniziert. Es erweist sich jedoch als vorteilhaft, wenn im Bereich des dem Unterdruckverband zugewandten Längsabschnitts des Leitungsmittels mehrere Öffnungen, insbesondere wenigstens zwei Öffnungen je cm Länge des Leitungsmittels vorgesehen sind. Hierbei erweist es sich auch als vorteilhaft, wenn die lichte Öffnungsfläche der Öffnungen in dem Leitungsmittel 5 bis 50% der Fläche der Ausnehmung in dem Trägermittel beträgt.

Die dem Unterdruckverband zugewandte und mit einer Kleberbeschichtung versehene Seite der Anschlussvorrichtung ist vorzugsweise vollständig, also auch über den Bereich der Ausnehmung, mit einer abziehbaren Release-Schicht versehen, die unmittelbar vor dem Applizieren der Anschlussvorrichtung auf den Unterdruckverband abgelöst wird.

Sämtliche vorausgehend beschriebenen Merkmale werden jeweils für sich und in beliebiger Kombination untereinander und mit weiteren Merkmalen als erfindungswesentlich betrachtet. Weitere Merkmale, Einzelheiten und Vorteile der Erfindung ergeben sich aus den beigefügten Patentansprüchen und aus der zeichnerischen Darstellung und nachfolgenden Beschreibung einer bevorzugten Ausführungsform der Erfindung.

In der Zeichnung zeigt:
- Figur 1: eine perspektivische Ansicht einer erfindungsgemäßen Anschlussvorrichtung zur Verwendung bei der Unterdruckbehandlung von Wunden;
- Figur 2: eine schematische nicht maßstabsgetreue Schnittansicht der Anschlussvorrichtung nach Figur 1; und
- Figuren 3a bis c: Schnittansichten von Leitungsmitteln der erfindungsgemäßen Anschlussvorrichtung (Schnittebene senkrecht zur Längserstreckung).

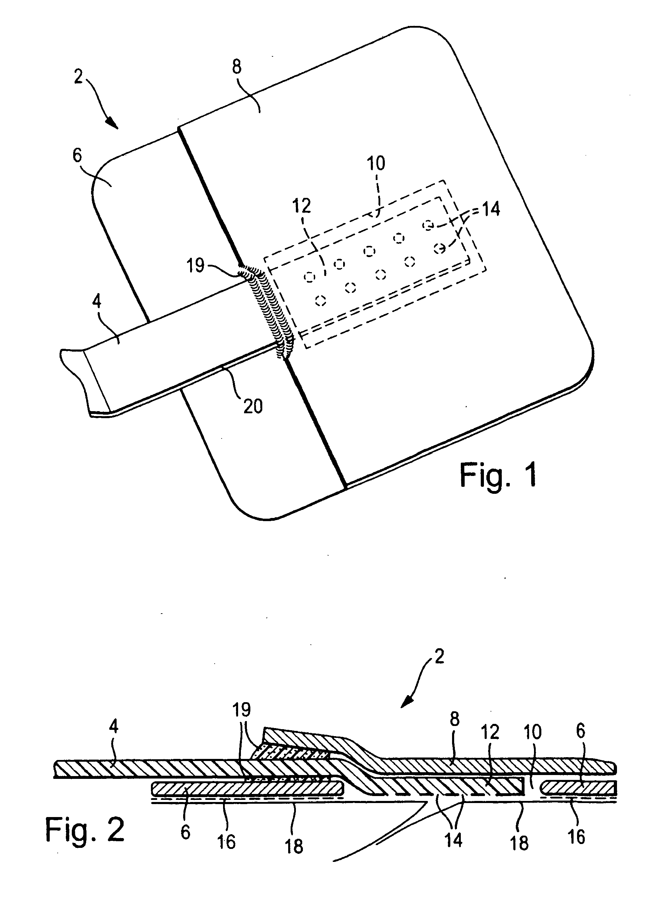

Die Figuren 1 und 2 zeigen verschiedene Ansichten einer erfindungsgemäßen insgesamt mit dem Bezugszeichen 2 bezeichneten Anschlussvorrichtung zur Verwendung bei der Unterdruckbehandlung von Wunden. Die dargestellte Anschlussvorrichtung 2 wird auf eine wundabgewandte Oberseite eines nicht dargestellten Unterdruckverbands, welcher eine zu behandelnde Wunde überfängt und zur Atmosphäre hin unterdruckdicht abschließt, vorzugsweise lösbar aufgebracht.

Die Anschlussvorrichtung 2 umfasst ein flaches Leitungsmittel 4 aus einem elastomeren biegsamen Material und ein flächenhaft erstrecktes erstes Trägermittel 6 und ein flächenhaft erstrecktes zweites Trägermittel 8, zwischen denen das flache Leitungsmittel 4 sandwichartig aufgenommen ist, wobei die beiden flächenhaften Trägermittel 6, 8 das flache Leitungsmittel 4 halten und stützen. Auf das Leitungsmittel 4 ausgeübte Druck- oder Biegekräfte oder Verwindungen werden somit gleichmäßig in den Verbund aus den beiden einander wenigstens teilweise überlagernden Trägermittel 6, 8 eingeleitet und von diesem aufgenommen.

Wie sich am besten aus Figur 2 ersehen lässt, ist in dem ersten unteren Trägermittel 6 eine ausladende Ausnehmung 10 ausgebildet, deren Breite ungefähr der Breite des Leitungsmittels 4 entspricht, so dass sich das Leitungsmittel 4 flächenhaft in diese Ausnehmung 10 hineinerstrecken kann. In diesem Bereich ist somit das Leitungsmittel 4 mit einem Längsabschnitt 12 unmittelbar dem Unterdruckverband zugewandt. In diesem Längsabschnitt 12 der dem Unterdruckverband zugewandten Seitenwandung des Leitungsmittels 4 sind vorzugsweise mehrere Öffnungen 14 ausgebildet, die mit Öffnungen, Einschnitten oder Schlitzen in dem nicht dargestellten Unterdruckverband kommunizieren.

Die dem Unterdruckverband zugewandte Unterseite des ersten Trägermittels 6 ist entweder mit einer angedeuteten Haftkleberbeschichtung 16 versehen oder sie ist selbsthaftend ausgebildet. Die gesamte Unterseite einschließlich der Ausnehmung 10 ist von einer ablösbaren zweiteiligen Release-Folie 18 abgedeckt, die kurz vor dem Applizieren der Anschlussvorrichtung 2 auf den nicht dargestellten Unterdruckverband abgelöst wird.

Im beispielhaft dargestellten Fall ist die Ausnehmung 10 in dem ersten Trägermittel 6 rechteckförmig ausgebildet, so dass sich das Leitungsmittel 4 flächenhaft und komplementär hineinerstrecken kann. Das erste Trägermittel 6 ist von dem zweiten Trägermittel 8 überfangen, derart, dass eine möglichst große Anlagefläche bzw. ein großer flächenhafter Kontaktbereich zwischen dem ersten und dem zweiten Trägermittel 6, 8 um die Ausnehmung 10 herum resultiert. Das obere Trägermittel 8 ist jedoch gegenüber dem unteren Trägermittel 6 im Ausmündungsbereich des Leitungsmittels 4 zurückgesetzt. Zwischen dem flachen Leitungsmittel 4 und den beiden Trägermitteln 6 und 8 wird eine unterdruckdichte Laminatverbindung ausgebildet. Zusätzlich ist im Ausmündungsbereich des Leitungsmittels 4 zwischen den beiden Trägermitteln eine Dichtmasse 19 vorgesehen. Bei der Dichtmasse 19 handelt es sich beispielhaft um einen Reaktiv-Klebstoff, insbesondere einen Silikonkleber.

Die beiden Trägermittel 6, 8 sind bei diesem Ausführungsbeispiel von Hydrokolloidschichten gebildet, die vorzugsweise selbsthaftend sind, so dass die oben erwähnte zusätzliche Haftkleberbeschichtung 16 nicht benötigt wird. Es können aber auch andere Materialien für die Trägermittel verwendet werden.

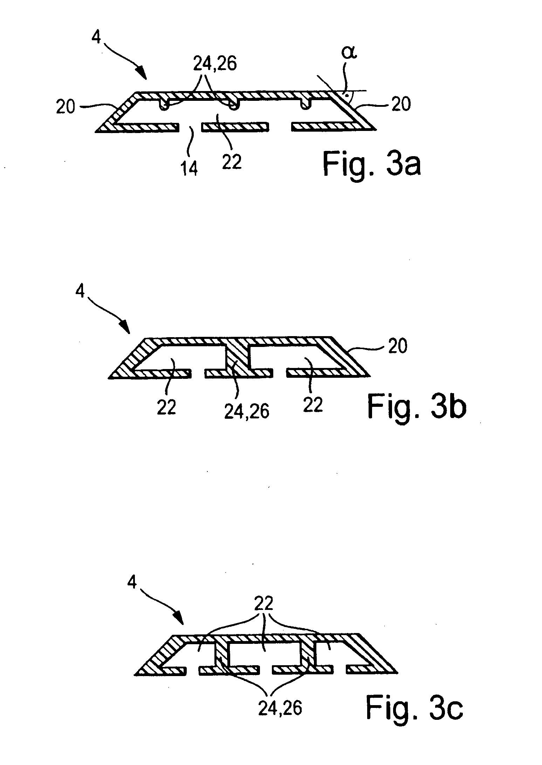

Die Ausbildung des Leitungsmittels 4 ist anhand verschiedener Ausführungsformen in den Figuren 3a bis c dargestellt. Das Leitungsmittel 4 ist nach einer bevorzugten Ausführungsform der Anschlussvorrichtung 2 trapezförmig ausgebildet und umfasst entlang seiner Längserstreckung zwei schräg in Richtung auf das erste Trägermittel 6 abfallende Flanken 20, deren Winkel α zur Erstreckungsebene des Leitungsmittels 4 bzw. des Trägermittels 6 ca. 40 bis 50° beträgt. Bei dem Leitungsmittel 4 gemäß Figur 3a ist ein einziger Kanal 22 ausgebildet, der mit Unterdruck beaufschlagbar ist. Bei den alternativen Ausführungsformen nach Figuren 3b und 3c sind zwei bzw. drei Kanäle 22 ausgebildet, wobei der eine Kanal der Figur 3b oder die beiden äußeren kleineren Kanäle gemäß Figur 3c als Spülleitung zur Heranführung eines Spülmediums dienen können.

Das flachbauende und aus einem biegsamen Material, vorzugsweise auf Silikonbasis, und weiter vorzugsweise einstückig ausgebildete Leitungsmittel 4 ist an seiner Innenseite mit Mitteln 24 zur Verhinderung des Kollabierens des Leitungsmittels 4 ausgebildet. Diese Mittel 24 sind von in Längsrichtung durchgehenden Rippen 26 gebildet, die einstückig mit dem Leitungsmittel 4 hergestellt sind. Bei der Ausführungsform des Leitungsmittels 4 gemäß Figuren 3b und 3c werden diese Mittel 28 von den Wänden (Septen) zwischen den Kanälen 22 gebildet.

In Figur 2 ist noch die Dickenstreckung D des Verbunds aus Leitungsmittel 4 und Trägermitteln 6, 8 angedeutet. Sie beträgt vorzugsweise höchstens 7 mm, insbesondere höchstens 5 mm und weiter vorzugsweise lediglich 2 bis 4 mm.

## Patentansprüche

1. Anschlussvorrichtung (2) zur Verwendung bei der Unterdruckbehandlung von Wunden, mit einem mit Unterdruck beaufschlagbaren Leitungsmittel (4) und mit einem flächenhaften unterdruckdichten ersten Trägermittel (6) für das Leitungsmittel (4), an dem das Leitungsmittel (4) unterdruckdicht gehalten ist, wobei das Trägermittel (6) auf einen die Wunde überfangenden und gegen die Atmosphäre dichtend abschließenden Unterdruckverband aufbringbar ist, wobei das Leitungsmittel (4) biegsam und flach ausgebildet ist und einen Querschnitt aufweist, dessen Breitenerstreckung wesentlich größer als seine Dickenerstreckung ist, und wobei das Leitungsmittel (4) einen Längsabschnitt (12) aufweist, dessen Wandung wenigstens eine Öffnung (14) aufweist, und wobei in dem ersten Trägermittel (6) eine rechteckförmige ausladende Ausnehmung (10) ausgebildet ist, deren Breite wenigstens so groß wie die Breite des Leitungsmittels (4) ist und deren Länge wenigstens 15 mm beträgt, so dass sich der Längsabschnitt (12) des Leitungsmittels (4) flächenhaft und komplementär in die Ausnehmung (10) hineinerstreckt, und der Längsabschnitt (12) und die wenigstens eine Öffnung (14) in dem Längsabschnitt (12) bei Aufbringung der Anschlussvorrichtung auf den Unterdruckverband unmittelbar ohne Zwischenordnung weiterer Materialien dem Unterdruckverband zugewandt sind, so dass die wenigstens eine Öffnung (14) in dem Längsabschnitt (12) des Leitungsmittels (4) direkt mit wenigstens einer Öffnung in dem Unterdruckverband und durch diese hindurch mit dem Wundraum kommuniziert, und dass das Leitungsmittel (4) dadurch unterdruckdicht an dem ersten Trägermittel (6) gehalten ist, dass es von einem zweiten flächenhaften Trägermittel (8) überfangen und so zwischen dem ersten und dem zweiten Trägermittel (6, 8) sandwichartig aufgenommen ist, wobei das zweite Trägermittel (8) in der Projektion auf die Ebene des ersten Trägermittels (6) die Ausnehmung (10) vollständig überfängt.

2. Anschlussvorrichtung nach Anspruch 1, **dadurch gekennzeichnet, dass** die Länge der Ausnehmung (10) des Trägermittels (6) wenigstens 20 mm beträgt.

3. Anschlussvorrichtung nach Anspruch 1 oder 2, **dadurch gekennzeichnet, dass** das erste Trägermittel (6) auf seiner dem Unterdruckverband zugewandten Seite eine Kleberbeschichtung (16) trägt oder selbsthaftend ausgebildet ist, so dass die Anschlussvorrichtung direkt auf eine wundabgewandte Oberseite des Unterdruckverbands haftend und unterdruckdicht applizierbar ist.

4. Anschlussvorrichtung nach Anspruch 1, 2 oder 3, **dadurch gekennzeichnet, dass** das zweite Trägermittel (8) in Bezug auf die Erstreckung des ersten Trägermitteln (6) im Bereich der Ausmündung des Leitungsmittels (4) zurückgesetzt ist, so dass dort ein überlappungsfreier Bereich des ersten Trägermittels (6) gebildet ist.

5. Anschlussvorrichtung nach einem oder mehreren der vorstehenden Ansprüche, **dadurch gekennzeichnet, dass** zum Abdichten zwischen dem ersten und dem zweiten Trägermittel (6, 8) eine Dichtmasse (19) verwandt ist, die zugleich haftvermittelnd zwischen dem ersten und dem zweiten Trägermittel (6, 8) wirken kann.

6. Anschlussvorrichtung nach einem oder mehreren der vorstehenden Ansprüche, **dadurch gekennzeichnet, dass** das erste und/oder das zweite Trägermittel (6, 8) aus einem thermoplastischen Elastomer gebildet ist oder solche Materialien umfasst.

7. Anschlussvorrichtung nach einem oder mehreren der vorstehenden Ansprüche, **dadurch gekennzeichnet, dass** das elastomere Material, aus dem das Leitungsmittel (4) gebildet ist, eine Shore A - Härte von höchstens 60 aufweist.

8. Anschlussvorrichtung nach einem oder mehreren der vorstehenden Ansprüche, **dadurch gekennzeichnet, dass** die Dickenerstreckung (D) des Verbunds aus Leitungsmittel (4) und Trägermittel(n) (6, 8) höchstens 10 mm beträgt.

9. Anschlussvorrichtung nach einem oder mehreren der vorstehenden Ansprüche, **dadurch gekennzeichnet, dass** die Breitenerstreckung des Leitungsmittels (4) wenigstens 10 mm beträgt.

10. Anschlussvorrichtung nach einem oder mehreren der vorstehenden Ansprüche, **dadurch gekennzeichnet, dass** das Leitungsmittel (4) im Inneren angeformte Mittel (24) zur Verhinderung des Kollabierens des Leitungsmittels (4) bei Unterdruckbeaufschlagung aufweist und
dass die Mittel (24) zur Verhinderung des Kollabierens von Rippen (26) oder Vorsprüngen gebildet sind.

11. Anschlussvorrichtung nach einem oder mehreren der vorstehenden Ansprüche, **dadurch gekennzeichnet, dass** das Leitungsmittel (4) mehrere voneinander druckdicht abgetrennte Kanäle (22) umfasst.

12. Anschlussvorrichtung nach einem oder mehreren der vorstehenden Ansprüche, **dadurch gekennzeichnet, dass** das flächenhafte erste und/oder zweite Trägermittel (6, 8) aus einem biegsamen elastomeren Material einer Shore A - Härte von 5 - 60 gebildet ist.

13. Anschlussvorrichtung nach einem oder mehreren der vorstehenden Ansprüche, **dadurch gekennzeichnet, dass** die flächenhafte Erstreckung des ersten Trägermittels (6) einschließlich seiner Ausnehmung (10) wenigstens das 1,5-fache der senkrecht auf das Trägermittel (6) projizierten Fläche des Leitungsmittels (4) beträgt.

14. Anschlussvorrichtung nach einem oder mehreren der vorstehenden Ansprüche, **dadurch gekennzeichnet, dass** das Leitungsmittel (4) im Querschnitt betrachtet trapezförmig ausgebildet ist.

15. Anschlussvorrichtung nach einem oder mehreren der vorstehenden Ansprüche, **dadurch gekennzeichnet, dass** die lichte Öffnungsfläche der Öffnungen (14) in dem. Leitungsmittel 5 - 50 % der Fläche der Ausnehmung (10) beträgt.

## Claims

1. Connecting device (2) for use for the treatment of wounds with vacuum, comprising a conduit means (4) which can be loaded with vacuum, and a flat vacuum-tight first carrier means (6) for the conduit means (4) on which the conduit means (4) is held in a vacuum-tight fashion, wherein the carrier means (6) can be disposed onto a vacuum dressing which extends over the wound and tightly seals it from atmosphere, wherein the conduit means (4) is flexible and flat and has a crosssection wherein the width of the crossection is substantially larger than its thickness and wherein the conduit means (4) has a longitudinal section (12) the wall of which has at least one opening (14) and wherein a broad rectangular recess (10) is formed in the first carrier means (6) the width of which is at least as large as the width of the conduit means (4) and the length of which is at least 15 mm, so that the longitudinal section (12) of the conduit means (4) flatly and complementary extends into this recess (10) and wherein the longitudinal section (12) and the at least one opening (14) in the longitudinal section (12) upon attachment of the connecting device onto the vacuum dressing directly face the vacuum dressing without interposition of any further materials, so that the at least one opening (14) in the longitudinal section (12) of the conduit means (4) directly communicates with the at least one opening in the vacuum dressing and therethrough with the wound space, and wherein the conduit means (4) is held on the first carrier means (6) in a vacuum-tight fashion in that a second extensive carrier means (8) extends over it and thereby receives it between the first and the second carrier means (6, 8) like a sandwich, wherein the second carrier means (8) completely extends over the recess (10) in the projection onto the plane of the first carrier means (6).

2. Connecting device according to claim 1, **characterized in that** the length of the recess (10) of the carrier means (6) is at least 20 mm.

3. Connecting device according to claim 1 or 2, **characterized in that** the first carrier means (6) has an adhesive coating (16) or is selfadherent on its side facing the vacuum dressing, such that the connecting device can be directly applied onto an upper side of the vacuum dressing facing away from the wound in an adhesive and vacuum-tight fashion.

4. Connecting device according to claim 1, 2 or 3, **characterized in that** the second carrier means (8) is offset with respect to the extension of the first carrier means (6) in the area of the outlet of the conduit means (4) such that a non-overlapping area of the carrier means (6) is formed at that location.

5. Connecting device according to any one or more of the preceding claims, **characterized in that** a sealing agent (19) is used for sealing between the first and the second carrier means (6, 8), which simultaneously promotes adhesion between the first and the second carrier means (6, 8).

6. Connecting device according to any one or more of the preceding claims, **characterized in that** the first and/or the second carrier means (6, 8) are formed from a thermoplastic elastomer or comprise such materials.

7. Connecting device according to any one or more of the preceding claims, **characterized in that** the elastomeric material from which the conduit means (4) is formed has a Shore A - hardness of at most 60.

8. Connecting device according to any one or more of the preceding claims, **characterized in that** the extension in thickness (D) of the composite of conduit means (4) and carrier means (6, 8) is at most 10 mm.

9. Connecting device according to any one or more of the preceding claims, **characterized in that** the extension in width of the conduit means (4) is at least 10 mm.

10. Connecting device according to any one or more of the preceding claims, **characterized in that** the conduit means (4) comprises means (24) which are formed on the inside, for preventing collapse of the conduit means (4) upon vacuum load and that the means (24) for preventing collapse are formed by ribs (26) or projections.

11. Connecting device according to any one or more of the preceding claims, **characterized in that** the conduit means (4) comprises several channels (22) which are separated from each other in a vacuum-tight fashion.

12. Connecting device according to any one or more of the preceding claims, **characterized in that** the flat first and/or second carrier means (6, 8) is/are formed from a flexible elastomeric material of a Shore A - hardness of 5 to 60.

13. Connecting device according to any one or more of the preceding claims, **characterized in that** the flat extension of the first carrier means (6) including its recess (10) is at least 1.5 times the area of the conduit means (4) projected perpendicularly onto the carrier means (6).

14. Connecting device according to any one or more of the preceding claims, **characterized in that** the conduit means (4) has a trapezoidal cross-section.

15. Connecting device according to any one or more of the preceding claims, **characterized in that** the clearance area of the openings (14) in the conduit means is 5 to 50 % of the surface of the recess (10).

## Revendications

1. Dispositif de raccordement (2) utilisé pour le traitement par pression négative de plaies, comprenant un moyen de conduite (4) apte à être soumis à une pression négative ainsi qu'un premier moyen support (6) en nappe pour ledit moyen de conduite (4), qui est étanche à la pression négative et sur lequel ledit moyen de conduite (4) est maintenu d'une manière étanche à la pression négative, ledit moyen support (6) pouvant être appliqué sur un pansement à pression négative recouvrant la plaie et fermant celle-ci de façon étanche par rapport à l'atmosphère, ledit moyen de conduite (4) étant réalisé de manière flexible et plate et présentant une section transversale dont l'extension en largeur est sensiblement plus grande que son extension en épaisseur, et ledit moyen de conduite (4) comprenant une portion longitudinale (12) dont la paroi présente au moins une ouverture (14), et dans ledit premier moyen support (6) étant ménagé un évidement (10) rectangulaire débordant dont la largeur est au moins égale à la largeur du moyen de conduite (4) et dont la longueur est de 15 mm au moins, de sorte que ladite portion longitudinale (12) du moyen de conduite (4) s'étend en nappe et complémentairement dans ledit évidement (10), et, lorsque ledit dispositif de raccordement est appliqué sur le pansement à pression négative, ladite portion longitudinale (12) et ladite au moins une ouverture (14) dans la portion longitudinale (12) étant tournées vers le pansement à pression négative sans interposition d'autres matières, de sorte que ladite au moins une ouverture (14) dans la portion longitudinale (12) du moyen de conduite (4) communique directement avec au moins une ouverture dans ledit pansement à pression négative et, à travers celle-ci, avec la zone de la plaie, et que, grâce à cela, ledit moyen de conduite (4) est maintenu d'une manière étanche à la pression négative sur ledit premier moyen support (6), qu'il est recouvert d'un deuxième moyen support (8) en nappe et est ainsi reçu en sandwich entre lesdits premier et deuxième moyens supports (6, 8), le deuxième moyen support (8) recouvrant, dans la projection sur le plan du premier moyen support (6), complètement ledit évidement (10).

2. Dispositif de raccordement selon la revendication 1, **caractérisé par le fait que** la longueur de l'évidement (10) du moyen support (6) est de 20 mm au moins.

3. Dispositif de raccordement selon la revendication 1 ou 2, **caractérisé par le fait que** ledit premier moyen support (6) présente, sur sa face montrant vers le pansement à pression négative, un revêtement de colle (16) ou est réalisé de manière auto-adhésive de sorte que le dispositif de raccordement puisse être appliqué de manière adhésive et étanche à la pression négative, directement sur une face supérieure du pansement à pression négative, qui est opposée à la plaie.

4. Dispositif de raccordement selon la revendication 1, 2 ou 3, **caractérisé par le fait que** ledit deuxième moyen support (8) est en retrait par rapport à l'extension du premier moyen support (6), dans la zone où débouche ledit moyen de conduite (4), de manière à y former une zone exempte de chevauchement du premier moyen support (6).

5. Dispositif de raccordement selon l'une ou plusieurs des revendications précédentes, **caractérisé par le fait que**, pour l'étanchement entre les premier et deuxième moyens supports (6, 8), on utilise un matériau d'étanchéité (19) qui peut agir en même temps en tant qu'agent adhésif entre les premier et deuxième moyens supports (6, 8).

6. Dispositif de raccordement selon l'une ou plusieurs des revendications précédentes, **caractérisé par le fait que** ledit premier et/ou ledit deuxième moyens supports (6, 8) est réalisé dans un élastomère thermoplastique ou comprend de telles matières.

7. Dispositif de raccordement selon l'une ou plusieurs des revendications précédentes, **caractérisé par le fait que** la matière élastomère à partir de laquelle est réalisé ledit moyen de conduite (4) présente une dureté Shore A de 60 tout au plus.

8. Dispositif de raccordement selon l'une ou plusieurs des revendications précédentes, **caractérisé par le fait que** l'extension en épaisseur (D) de l'ensemble constitué par le moyen de conduite (4) et le(s) moyen(s) support(s) (6, 8) est de 10 mm tout au plus.

9. Dispositif de raccordement selon l'une ou plusieurs des revendications précédentes, **caractérisé par le fait que** l'extension en largeur du moyen de conduite (4) est de 10 mm au moins.

10. Dispositif de raccordement selon l'une ou plusieurs des revendications précédentes, **caractérisé par le fait que** ledit moyen de conduite (4) présente des moyens (24) qui sont surmoulés à l'intérieur et destinés à éviter que le moyen de conduite (4) ne subisse un collapsus lorsqu'il est soumis à la pression négative, et que lesdits moyens (24) destinés à éviter un collapsus sont constitués par des nervures (26) ou projections.

11. Dispositif de raccordement selon l'une ou plusieurs des revendications précédentes, **caractérisé par le fait que** ledit moyen de conduite (4) comprend une pluralité de canaux (22) isolés les uns des autres d'une manière étanche à la pression.

12. Dispositif de raccordement selon l'une ou plusieurs des revendications précédentes, **caractérisé par le fait que** le premier et/ou le deuxième moyens supports (6, 8) en nappe est réalisé dans une matière flexible élastomère ayant une dureté Shore A comprise entre 5 et 60.

13. Dispositif de raccordement selon l'une ou plusieurs des revendications précédentes, **caractérisé par le fait que** l'extension en nappe du premier moyen support (6) y compris son évidement (10) est au moins 1,5 fois la surface du moyen de conduite (4) projetée perpendiculairement sur le moyen support (6).

14. Dispositif de raccordement selon l'une ou plusieurs des revendications précédentes, **caractérisé par le fait que**, vu en coupe transversale, ledit moyen de conduite (4) est trapézoïdal.

15. Dispositif de raccordement selon l'une ou plusieurs des revendications précédentes, **caractérisé par le fait que** la surface libre d'ouverture des ouvertures (14) dans le moyen de conduite est comprise entre 5 et 50 % de la surface de l'évidement (10).
